# EUROPEAN PATENT APPLICATION

(11) **EP 1 132 087 A1**
(43) Date of publication of application: **12.09.2001**
(21) Application number: 99972100.4
(22) Date of filing: 10.11.1999
(51) Int. Cl.: A61K 31/404, A61K 31/415, A61P 15/00, C07D 209/08, C07D 209/10, C07D 235/08

(54) **REMEDIES FOR POLYCYSTIC OVARY SYNDROME**

(30) Priority: 13.11.1998 JP 32402698
(71) Applicant: FUJISAWA PHARMACEUTICAL CO., LTD., Osaka-shi Osaka 541-8514 (JP); Daicel Chemical Industries, Ltd., Osaka 590-8501 (JP)
(72) Inventor: HIROSUMI, Jiro, Amagasaki-shi, Hyogo 661-0033 (JP); MUTOH, Seitaro, Tsukuba-shi, Ibaraki 305-0053 (JP); YAMASAKI, Noritsugu, Shikama-ku, Himeji-shi, Hyogo 672-8071 (JP); IMOTO, Takafumi, Arai-shi, Niigata 944-0041 (JP)
(74) Representative: Gille Hrabal Struck Neidlein Prop Roos
(86) International application number: PCT/JP99/06267
(87) International publication number: WO 00/28991

(57) **Abstract**

The present invention relates to a therapeutic agent for polycystic ovary syndrome, which comprises a compound of the following formula (1) or (2), or a pharmaceutically acceptable salt thereof as an active ingredient: 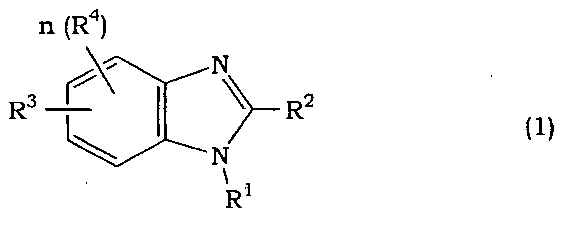 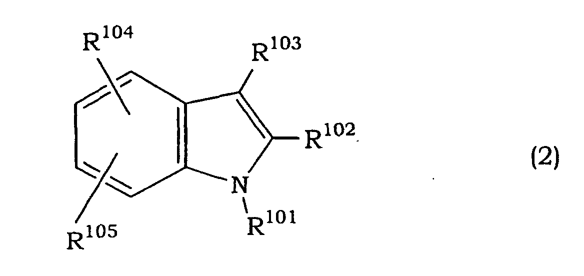 wherein each symbol is as defined in the specification.

## Description

### Technical Field

The present invention relates to a therapeutic agent for polycystic ovary syndrome. More particularly, the present invention relates to a therapeutic agent for polycystic ovary syndrome, which contains a specific compound or a pharmaceutically acceptable salt thereof as an active ingredient.

### Background Art

The polycystic ovary syndrome refers to the disease state accompanying polycystic ovary on both sides, ovulation disorder, difficult menstruation and infertility, obesity, sterility, hypertrichosis, acne, alopecia, defemination and the like. This syndrome means an endocrine disease that 5 - 10% of premenopausal women are considered to develop, with expected patients in a large number. This syndrome is characterized by hyperandrogenism, and oligo- or anovulation due to a gonadotrophin secretion disorder. It is considered that insulin resistance is involved in the onset of the polycystic ovary syndrome, and therefore, women having the syndrome have a high risk of developing non-insulin dependent diabetes.

### Disclosure of the Invention

The present invention aims at providing a therapeutic agent for polycystic ovary syndrome, which contains a specific compound or a pharmaceutically acceptable salt thereof as an active ingredient.

Accordingly, the present invention relates to a therapeutic agent for polycystic ovary syndrome, which contains a compound of the following formula (1) or (2), or a pharmaceutically acceptable salt thereof as an active ingredient. 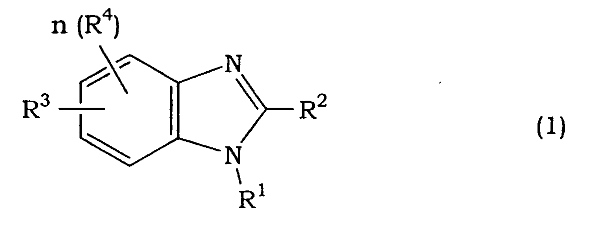 wherein
- R¹: is hydrogen atom, arylsulfonyl or lower alkyl wherein lower alkyl may be substituted by one or two group(s) from aryl optionally substituted by a group selected from the group consisting of halogen atom, haloaryl, lower alkyl, halo(lower)alkyl, lower alkoxy, nitro, amino, cyano, aryl, aryl(lower)alkyl, aryl(lower)alkoxy, haloaryl(lower)alkoxy, arylsulfonyl(lower)alkyl, arylsulfonylamino, cyanoaryl and heterocyclic ring, and heterocyclic ring,
- R²: is hydrogen atom, lower cycloalkyl, hydroxy, lower alkoxy, mercapto, lower alkylthio, amino, lower alkylamino, carboxy, aryl or lower alkyl, wherein lower alkyl may be substituted by halogen atom, lower alkoxy, cyano, chlorocarbonyl, aryl or heterocyclic ring,
- R³: is carboxy, esterified carboxy, amidated carboxy, amino, amide or sulfonyl, wherein amino and amide are each optionally substituted by acyl or sulfonyl, said sulfonyl being bonded with halogen atom, amino or acylamino and R³ may be bonded to the skeleton via lower alkylene or lower alkenylene, and
- R⁴: is a neutral substituent, and
- n: is an integer of 0 - 3; 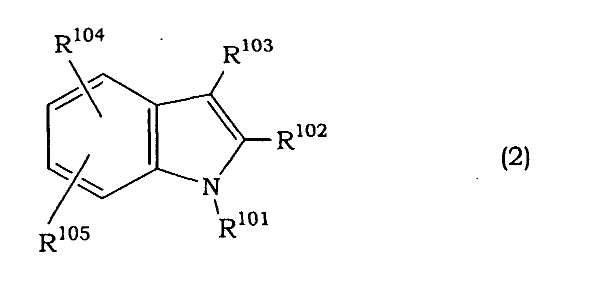
wherein
- R¹⁰¹ - R¹⁰³: are each
1) hydrogen atom,
2) lower alkyl, lower alkylthio or lower alkoxy(lower)alkyl or
3) lower alkyl, oxy, oxy(lower)alkyl, lower alkoxy, carbonyl, lower alkenyl, optionally substituted imino, lower alkylimino wherein nitrogen atom is optionally substituted, thio(lower)alkyl or lower alkylthio, wherein each group is bonded with aryl or heterocyclic ring or substituted with aryl or heterocyclic ring, said aryl and heterocyclic ring each being optionally substituted by halogen atom; nitro; lower alkylamino; acylamino; lower alkyl; lower alkoxy; halo(lower)alkyl; lower cycloalkyl; or aryl, heterocyclic ring, aryl(lower)alkyl, heterocyclic lower alkyl, aryl(lower)alkoxy, heterocyclic lower alkoxy, aryl(lower)alkenyl or heterocyclic lower alkenyl, each being optionally substituted by halogen atom or lower alkyl,
provided that R¹⁰¹ - R¹⁰³ are not hydrogen atoms at the same time,
- R¹⁰⁴: is hydrogen atom or lower alkyl, and
- R¹⁰⁵: is carboxy, esterified carboxy or amidated carboxy.

These compounds are known as, for example, insulin sensitivity enhancers, and described in, for example, International Publication Nos. WO97/24334, WO98/15530 and the like.

In the above and the following description in this specification, preferable examples and the detail of various definitions encompassed in the present invention are explained in the following. _

By the lower is meant the carbon number of 8 or less, preferably 6 or less, unless particularly indicated.

By the lower alkyl is meant linear or branched alkyl such as methyl, ethyl, n-propyl, i-propyl, n-butyl, i-butyl, sec-butyl, t-butyl, n-pentyl, i-pentyl, sec-pentyl, t-pentyl, 2-methylbutyl, n-hexyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 2,2-dimethylbutyl, 3,3-dimethylbutyl, 1-ethyl-1-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, 4-ethylpentyl, 1,1-dimethylpentyl, 2,2-dimethylpentyl, 3,3-dimethylpentyl, 4,4-dimethylpentyl, 1-propylbutyl, n-octyl, 1-methylheptyl, 2-methylheptyl, 3-methylheptyl, 4-methylheptyl, 5-methylheptyl, 6-methylheptyl, 1-ethylhexyl, 2-ethylhexyl, 3-ethylhexyl, 4-ethylhexyl, 5-ethylhexyl, 1,1-dimethylhexyl, 2,2-dimethylhexyl, 3,3-dimethylhexyl, 4,4-dimethylhexyl, 5,5-dimethylhexyl, 1-propylpentyl, 2-propylpentyl and the like, preferably those having 3 to 6 carbon atoms.

The lower alkylene is alkylene preferably having 6 or less, particularly preferably 1 to 3, carbon atoms, such as methylene, ethylene, propylene, butylene, pentylene, hexylene and the like. The lower alkenylene is alkenylene preferably having 6 or less, particularly preferably 2 or 3, carbon atoms, such as ethenylene, 1-propenylene, 2-propenylene, 1-butenylene, 2-butenylene, 3-butenylene, 1-pentenylene, 2-pentenylene, 3-pentenylene, 4-pentenylene, 1-hexenylene, 2-hexenylene, 3-hexenylene, 4-hexenylene, 5-hexenylene and the like.

Examples of preferable lower alkenyl include linear or branched alkenyl, such as vinyl, 1-propenyl, 2-propenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 2-methyl-2-butenyl, 3-methyl-2-butenyl, 2-methyl-3-butenyl, 3-methyl-3-butenyl, 1-hexenyl, 2-hexenyl, 3-hexenyl, 4-hexenyl, 5-hexenyl, 2-methyl-1-pentenyl, 3-methyl-1-pentenyl, 4-methyl-1-pentenyl and the like, with preference given to those having 2 to 4 carbon atoms.

The halogen atom is exemplified by fluorine atom, chlorine atom, bromine atom and iodine atom, with preference given to fluorine atom, chlorine atom and bromine atom.

The halo(lower)alkyl is linear or branched alkyl having up to 8 carbon atoms, which is substituted by fluorine atom, chlorine atom, bromine atom or iodine atom, preferably linear or branched alkyl having up to 8 carbon atoms, more preferably 1 to 3 carbon atoms, which is substituted by fluorine atom, chlorine atom or bromine atom. Examples thereof include fluoromethyl, difluoromethyl, trifluoromethyl, chloromethyl, dichloromethyl, trichloromethyl, bromomethyl, dibromomethyl, tribromomethyl, 1-fluoroethyl, 1-chloroethyl, 1-bromoethyl, 2-fluoroethyl, 2-chloroethyl, 2-bromoethyl, 1,2-difluoroethyl, 1,2-dichloroethyl, 1,2-dibromoethyl, 2,2,2-trifluoroethyl, heptafluoroethyl, 1-fluoropropyl, 1-chloropropyl, 1-bromopropyl, 2-fluoropropyl, 2-chloropropyl, 2-bromopropyl, 3-fluoropropyl, 3-chloropropyl, 3-bromopropyl, 1,2-difluoropropyl, 1,2-dichloropropyl, 1,2-dibromopropyl, 2,3-difluoropropyl, 2,3-dichloropropyl, 2,3-dibromopropyl, 3,3,3-trifluoropropyl, 2,2,3,3,3-pentafluoropropyl, 2-fluorobutyl, 2-chlorobutyl, 2-bromobutyl, 4-fluorobutyl, 4-chlorobutyl, 4-bromobutyl, 4,4,4-trifluorobutyl, 2,2,3,3,4,4,4-heptafluorobutyl, perfluorobutyl, 2-fluoropentyl, 2-chloropentyl, 2-bromopentyl, 5-fluoropentyl, 5-chloropentyl, 5-bromopentyl, perfluoropentyl, 2-fluorohexyl, 2-chlorohexyl, 2-bromohexyl, 6-fluorohexyl, 6-chlorohexyl, 6-bromohexyl, perfluorohexyl, 2-fluoroheptyl, 2-chloroheptyl, 2-bromoheptyl, 7-fluoroheptyl, 7-chloroheptyl, 7-bromoheptyl, perfluoroheptyl and the like.

The oxy(lower)alkyl means lower alkyl substituted by oxy. The optionally substituted imino means imino that may be substituted by lower alkyl and the like.

The lower alkylimino having an optionally substituted nitrogen atom is imino bonded with lower alkyl, wherein the nitrogen atom may be substituted by lower alkyl and the like.

The thio(lower)alkyl means lower alkyl substituted by thio. The lower alkoxy is linear or branched alkoxy preferably having up to 6 carbon atoms. Examples thereof include methoxy, ethoxy, n-propyloxy, i-propyloxy, n-butyloxy, i-butyloxy, sec-butyloxy, t-butyloxy, n-pentyloxy, i-pentyloxy, sec-pentyloxy, t-pentyloxy, 2-methylbutoxy, n-hexyloxy, i-hexyloxy, t-hexyloxy, sec-hexyloxy, 2-methylpentyloxy, 3-methylpentyloxy, 1-ethylbutyloxy, 2-ethylbutyloxy, 1,1-dimethylbutyloxy, 2,2-dimethylbutyloxy, 3,3-dimethylbutyloxy, 1-ethyl-1-methylpropyloxy and the like. More preferably, it is methoxy, ethoxy, n-propyloxy, i-propyloxy, n-butyloxy, i-butyloxy, sec-butyloxy, t-butyloxy and the like, particularly preferably those having 1 to 3 carbon atoms.

The lower cycloalkyl is cycloalkyl having 3 to 7 carbon atoms, which is preferably cyclopropyl, cyclobutyl, cyclopentyl, cyclohexyl, cycloheptyl and the like, more preferably cyclopropyl, cyclobutyl, cyclopentyl and cyclohexyl.

The lower alkoxy(lower)alkyl is linear or branched alkyl having up to 8 carbon atoms, which is substituted by linear or branched alkoxy having up to 8 carbon atoms. Examples thereof include methoxymethyl, methoxyethyl, methoxypropyl, methoxybutyl, methoxypentyl, methoxyhexyl, methoxyheptyl, methoxyoctyl, ethoxymethyl, ethoxyethyl, ethoxybutyl, ethoxypentyl, ethoxyhexyl, ethoxyheptyl, ethoxyoctyl, propyloxymethyl, propyloxyethyl, propyloxypropyl, propyloxybutyl, propyloxypentyl, i-propyloxymethyl, i-propyloxyethyl, i-propyloxypropyl, i-propyloxybutyl, i-propyloxypentyl, butyloxymethyl, butyloxyethyl, butyloxypropyl, butyloxybutyl, 1-butyloxymethyl, 1-butyloxyethyl, 1-butyloxypropyl, 1-butyloxybutyl, sec-butyloxymethyl, sec-butyloxyethyl, sec-butyloxypropyl, sec-butyloxybutyl, t-butyloxymethyl, t-butyloxyethyl, t-butyloxypropyl, t-butyloxybutyl, pentyloxymethyl, pentyloxyethyl, pentyloxypropyl, pentyloxybutyl, hexyloxymethyl, hexyloxyethyl, hexyloxypropyl and the like, preferably alkyl having 1 or 2 carbon atoms, which is bonded with alkoxy having 1 or 2 carbon atoms.

The tri(lower)alkylsilyl(lower)alkyl is the aforementioned lower alkyl bonded with trimethylsilyl, triethylsilyl, tripropylsilyl and the like.

The lower alkylamino is linear or branched alkylamino preferably having up to 6 carbon atoms. Examples thereof include methylamino, ethylamino, n-propylamino, i-propylamino, n-butylamino, i-butylamino, sec-butylamino, t-butylamino, n-pentylamino, i-pentylamino, sec-pentylamino, t-pentylamino, 2-methylbutylamino, n-hexylamino, 1-methylpentylamino, 2-methylpentylamino, 3-methylpentylamino, 4-methylpentylamino, 1-ethylbutylamino, 2-ethylbutylamino, 3-ethylbutylamino, 1,1-dimethylbutylamino, 2,2-dimethylbutylamino, 3,3-dimethylbutylamino, 1-ethyl-1-methylpropylamino and the like. More preferably, alkylamino having 1 to 4 carbon atoms, such as methylamino, ethylamino, n-propylamino, i-propylamino, n-butylamino, i-butylamino, sec-butylamino and t-butylamino, are exemplified.

The lower alkylthio is linear or branched alkylthio preferably having up to 6 carbon atoms. Examples thereof include methylthio, ethylthio, n-propylthio, i-propylthio, n-butylthio, i-butylthio, sec-butylthio, t-butylthio, n-pentylthio, i-pentylthio, sec-pentylthio, t-pentylthio, 2-methylbutylthio, n-hexylthio, i-hexylthio, t-hexylthio, sec-hexylthio, 2-methylpentylthio, 3-methylpentylthio, 1-ethylbutylthio, 2-ethylbutylthio, 1,1-dimethylbutylthio, 2,2-dimethylbutylthio, 3,3-dimethylbutylthio, 1-ethyl-1-methylpropylthio and the like. More preferably, alkylthio having 1 to 4 carbon atoms, such as methylthio, ethylthio, n-propylthio, i-propylthio, n-butylthio, i-butylthio, sec-butylthio, t-butylthio and the like, are exemplified.

The lower alkylthio(lower)alkyl is linear or branched alkyl defined above having up to 8 carbon atoms, which is substituted by linear or branched alkylthio defined above having up to 8 carbon atoms.

The lower alkoxycarbonyl is linear or branched alkoxycarbonyl wherein the alkyl moiety preferably has up to 6 carbon atoms. Exemplified are methoxycarbonyl, ethoxycarbonyl, n-propyloxycarbonyl, i-propyloxycarbonyl, n-butyloxycarbonyl, i-butyloxycarbonyl, sec-butyloxycarbonyl, t-butyloxycarbonyl, n-pentyloxycarbonyl, i-pentyloxycarbonyl, sec-pentyloxycarbonyl, t-pentyloxycarbonyl, 2-methylbutyloxycarbonyl, n-hexyloxycarbonyl, i-hexyloxycarbonyl, t-hexyloxycarbonyl, sec-hexyloxycarbonyl, 2-methylpentyloxycarbonyl, 3-methylpentyloxycarbonyl, 1-ethylbutyloxycarbonyl, 2-ethylbutyloxycarbonyl, 1,1-dimethylbutyloxycarbonyl, 2,2-dimethylbutyloxycarbonyl, 3,3-dimethylbutyloxycarbonyl, 1-ethyl-1-methylpropyloxycarbonyl and the like. More preferably, carbonyl bonded with alkoxy having 1 to 4 carbon atoms, such as methoxycarbonyl, ethoxycarbonyl, n-propyloxycarbonyl, i-propyloxycarbonyl, n-butyloxycarbonyl, 1-butyloxycarbonyl, sec-butyloxycarbonyl, t-butyloxycarbonyl and the like, are exemplified.

The lower alkanoyl is linear or branched alkylcarbonyl wherein the alkyl moiety preferably has up to 6 carbon atoms. Examples thereof include methylcarbonyl, ethylcarbonyl, n-propylcarbonyl, i-propylcarbonyl, n-butylcarbonyl, i-butylcarbonyl, sec-butylcarbonyl, t-butylcarbonyl, n-pentylcarbonyl, i-pentylcarbonyl, sec-pentylcarbonyl, t-pentylcarbonyl, 2-methylbutylcarbonyl, n-hexylcarbonyl, i-hexylcarbonyl, t-hexylcarbonyl, sec-hexylcarbonyl, 2-methylpentylcarbonyl, 3-methylpentylcarbonyl, 1-ethylbutylcarbonyl, 2-ethylbutylcarbonyl, 1,1-dimethylbutylcarbonyl, 2,2-dimethylbutylcarbonyl, 3,3-dimethylbutylcarbonyl, 1-ethyl-1-methylpropylcarbonyl and the like. More preferably, it is carbonyl bonded with alkyl having 1 to 4 carbon atoms, such as methylcarbonyl, ethylcarbonyl, n-propylcarbonyl, i-propylcarbonyl, n-butylcarbonyl, i-butylcarbonyl, sec-butylcarbonyl, and t-butylcarbonyl and the like.

The lower alkanesulfonyl is linear or branched alkanesulfonyl wherein the alkyl moiety preferably has up to 6 carbon atoms, such as methanesulfonyl, ethanesulfonyl, 1-propanesulfonyl, 2-propanesulfonyl, 1-butanesulfonyl, 2-butanesulfonyl, 1,1-dimethylethanesulfonyl, 1-(2-methylpropane)-sulfonyl, 1-pentanesulfonyl, 2-pentanesulfonyl, 3-pentanesulfonyl, 1-(3-methylbutane)sulfonyl, 1,1-dimethylpropanesulfonyl, 1-hexanesulfonyl, 2-hexanesulfonyl, 3-hexanesulfonyl, 1-(2-methylpentane)sulfonyl, 1-(3-methylpentane)sulfonyl, 1-(4-methylpentane)sulfonyl, 2-ethylbutanesulfonyl, 3-ethylbutanesulfonyl, 1,1-dimethylbutanesulfonyl, 2,2-dimethylbutanesulfonyl, 3,3-dimethylbutanesulfonyl, 1-ethyl-1-methylpropanesulfonyl and the like. Particularly preferably, it is alkylsulfonyl having 1 to 4 carbon atoms.

The aryl throughout this specification includes those having 6 to 10 carbon atoms, such as phenyl, naphthyl and the like, wherein simple "naphthyl" includes 1-naphtyl and 2-naphtyl. In addition, the benzene ring and naphthalene ring thereof may be substituted by, for example, the aforementioned halogen atom, lower alkyl, cyano, nitro, trifluoromethyl and the like.

The haloaryl is the above-mentioned aryl substituted by halogen atom, which includes mono-, di- or trihalophenyl, mono-, di- or trihalonaphthyl and the like, wherein the halogen atom may be the same or different.

The cyanoaryl means the above-mentioned aryl substituted by cyano. Examples thereof include cyanophenyl, cyanonaphthyl and the like.

The arylsulfonyl includes sulfonyl bonded with the aforementioned aryl, such as benzenesulfonyl, toluenesulfonyl, naphthalenesulfonyl and the like.

The aryl(lower)alkyl means the aforementioned lower alkyl bonded with the aforementioned aryl, such as benzyl, 1-phenylethyl, 2-phenylethyl, phenylpropyl, phenylbutyl, phenylpentyl, phenylhexyl, naphthylmethyl, naphthylethyl, naphthylpropyl, naphthylbutyl, naphthylpentyl and naphthylhexyl.

The aryl(lower)alkoxy includes benzyloxy, 1-phenylethyloxy, 2-phenylethyloxy, phenylpropyloxy, phenylbutyloxy, phenylpentyloxy, phenylhexyloxy, naphthylmethyloxy, naphthylethyloxy, naphthylpropyloxy, naphthylbutyloxy, naphthylpentyloxy and the like, optionally having a substituent on the benzene ring and naphthalene ring thereof.

The haloaryl(lower)alkoxy means the above-mentioned aryl(lower)alkoxy substituted by halogen atom, such as mono- or dihalophenyl(lower)alkoxy and the like, wherein the halogen atom may be the same or different.

The arylsulfonyl(lower)alkyl means the aforementioned lower alkyl bonded with the aforementioned arylsulfonyl, such as benzenesulfonylmethyl, toluenesulfonylmethyl, naphthalenesulfonylmethyl and the like.

The arylsulfonylamino includes amino bonded with the aforementioned arylsulfonyl, such as benzenesulfonylamino, toluenesulfonylamino, naphthalenesulfonylamino and the like.

The aryloxy includes oxygen atom bonded with the aforementioned aryl, such as phenoxy, 1-naphthoxy, 2-naphthoxy and the like.

The arylcarbonyl includes carbonyl bonded with the aforementioned aryl, such as phenylcarbonyl, naphthylcarbonyl and the like.

The arylcarbonylamino includes amino bonded with the aforementioned arylcarbonyl, such as phenylcarbonylamino, naphthylcarbonylamino and the like.

The aryl(lower)alkenyl includes alkenyl preferably having 6 or less carbon atoms, which is substituted by the aforementioned aryl, such as phenylethenyl, naphthylethenyl and the like.

The heterocyclic ring is exemplified by the below mentioned, such as pyridyl, quinolyl, isoquinolyl, thiazolyl, thiadiazolyl, benzofuranyl, dibenzofuranyl, thianaphthalenyl, 1H-1,2,3-triazolyl, 1,2,4-triazolyl, tetrazolyl, furyl, thienyl, pyrrolyl, imidazolyl, pyrimidinyl, indolyl, benzimidazolyl and the like, which include those substituted by the aforementioned halogen atom or lower alkyl, such as haloisoquinolyl and methylisoquinolyl.

The heterocyclic lower alkyl means the aforementioned lower alkyl substituted by the aforementioned heterocyclic ring, such as pyridylmethyl and the like, the haloheterocyclic lower alkyl means the aforementioned heterocyclic lower alkyl wherein the heterocyclic ring is substituted by halogen, and the heterocyclic lower alkoxy means oxy with which heterocyclic lower alkyl is bonded. The heterocyclic lower alkenyl means lower alkenyl substituted by heterocyclic ring.

The heterocyclic lower alkylamino means amino substituted by the aforementioned heterocyclic lower alkyl, such as pyridylmethylamino and the like, and the heterocyclic lower alkylcarbamoyl means carbamoyl substituted by the aforementioned heterocyclic lower alkyl, such as pyridylmethylcarbamoyl and the like.

Simple "pyridyl" includes 2-pyridyl, 3-pyridyl and 4-pyridyl, with no limitation on the binding site. Similarly, the binding site of the heterocyclic ring is not limited.

The lower alkylenedioxybenzyl is, for example, methylenedioxybenzyl, ethylenedioxybenzyl, propylenedioxybenzyl and the like.

The preferable "heterocyclic ring" means saturated or unsaturated heteromonocyclic or heteropolycyclic ring having at least one hetero atom, such as oxygen atom, sulfur atom, nitrogen atom and the like.

More preferable examples are the below-mentioned heterocyclic rings.
- 7 to 12-membered, preferably 9 or 10-membered, unsaturated fused heterocyclic ring (preferably bicyclic) having 1 to 5 nitrogen atoms, such as indolyl, isoindolyl, indolizinyl, benzimidazolyl, quinolyl, isoquinolyl, indazolyl, benzotriazolyl, tetrazolopyridyl, tetrazolopyridazinyl (e.g., tetrazolo[1,5-b]pyridazinyl etc.), dihydrotriazolopyridazinyl and the like;
- 7 to 12-membered, preferably 9 or 10-membered, unsaturated fused heterocyclic ring (preferably bicyclic) having 1 to 3 sulfur atoms and an S,S-dioxide thereof, such as dithianaphthalenyl (e.g., 4H-1,3-dithianaphthalenyl, 1,4-dithianaphthalenyl etc.), benzothiophenyl and S,S-dioxide thereof (e.g., benzo[a]thiophenyl and S,S-dioxide thereof, benzo[b]thiophenyl and S,S-dioxide thereof etc.) and the like;
- 3 to 8-membered, preferably 5 or 6-membered, unsaturated heteromonocyclic ring having 1 to 4 nitrogen atoms, such as pyrrolyl, pyrrolinyl, imidazolyl, pyrazolyl, pyridyl and an N-oxide thereof, pyrimidyl, pyrazinyl, pyridazinyl, triazolyl (e.g., 4H-1,2,4-triazolyl, 1H-1,2,3-triazolyl, 2H-1,2,3-triazolyl etc.), tetrazolyl (e.g., 1H-tetrazolyl, 2H-tetrazolyl etc.), dihydrotriazinyl (e.g., 4,5-dihydro-1,2,4-triazinyl, 2,5-dihydro-1,2,4-triazinyl etc.);
- 3 to 8-membered, preferably 5 or 6-membered, saturated heteromonocyclic ring having 1 to 4 nitrogen atoms, such as azetidinyl, pyrrolidinyl, imidazolidinyl, piperidinyl, pyrazolidinyl, piperazinyl and the like;
- 7 to 12-membered, preferably 9 or 10-membered, unsaturated fused heterocyclic ring (preferably bicyclic) having 1 or 2 oxygen atoms and 1 to 3 nitrogen atoms, such as benzoxazolyl, benzoxadiazolyl and the like;
- 3 to 8-membered, preferably 5 or 6-membered, unsaturated heteromonocyclic ring having 1 or 2 oxygen atoms and 1 to 3 nitrogen atoms, such as oxazolyl, isoxazolyl, oxadiazolyl (e.g., 1,2,4-oxadiazolyl, 1,3,4-oxadiazolyl, 1,2,5-oxadiazolyl etc.) and the like;
- 3 to 8-membered, preferably 5 or 6-membered, saturated heteromonocyclic ring having 1 or 2 oxygen atoms and 1 to 3 nitrogen atoms, such as morpholinyl and the like;
- 7 to 12-membered, preferably 9 or 10-membered, unsaturated fused heterocyclic ring (preferably bicyclic) having 1 or 2 sulfur atoms and 1 to 3 nitrogen atoms, such as benzothiazolyl, benzothiaziazolyl and the like;
- 3 to 8-membered, preferably 5 or 6-membered, unsaturated heteromonocyclic ring having 1 or 2 sulfur atoms and 1 to 3 nitrogen atoms, such as 1,3-thiazolyl, 1,2-thiazolyl, thiaziazolyl (e.g., 1,2,4-thiaziazolyl, 1,3,4-thiaziazolyl, 1,2,5-thiaziazolyl, 1,2,3-thiaziazolyl etc.) and the like;
- 3 to 8-membered, preferably 5 or 6-membered, saturated heteromonocyclic ring having 1 or 2 sulfur atoms and 1 to 3 nitrogen atoms, such as thiazolidinyl and the like;
- 3 to 8-membered, preferably 5 or 6-membered, unsaturated heteromonocyclic ring having 1 sulfur atom, such as thienyl etc.; and the like.

The preferable "esterified carboxy" is exemplified by the following.

Examples of the preferable ester moiety of esterified carboxy include lower alkyl ester (e.g., methyl ester, ethyl ester, propyl ester, isopropyl ester, butyl ester, i-butyl ester, t-butyl ester, pentyl ester, hexyl ester and the like).

The lower alkyl ester may have at least one suitable substituent, and is exemplified by lower alkanoyloxy(lower)alkyl ester [e.g., acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(or 2-, 3- or 4-)acetoxybutyl ester, 1-(or 2-)propionyloxyethyl ester, 1-(or 2- or 3-)propionyloxypropyl ester, 1-(or 2-)butyryloxyethyl ester, 1-(or 2-)isobutyryloxyethyl ester, 1-(or 2-)pivaloyloxyethyl ester, 1-(or 2-)hexanoyloxyethyl ester, isobutyryloxymethyl ester, 2-ethylbutyryloxymethyl ester, 3,3-dimethylbutyryloxymethyl ester, 1-(or 2-)pentanoyloxyethyl ester and the like], lower alkanesulfonyl(lower)alkyl ester (e.g., 2-methylethyl ester and the like), mono(or di or tri)halo(lower)alkyl ester (e.g., 2-iodoethyl ester, 2,2,2-trichloroethyl ester and the like); lower alkoxycarbonyloxy(lower)alkyl ester [e.g., methoxycarbonyloxymethyl ester, ethoxycarbonyloxymethyl ester, propoxycarbonyloxymethyl ester, t-butoxycarbonyloxymethyl ester, 1-(or 2-)methoxycarbonyloxyethyl ester, 1-(or 2-)ethoxycarbonyloxyethyl ester, 1-(or 2-)isopropoxycarbonyloxyethyl ester and the like], phthalidylidene(lower)alkyl ester, and (5-lower alkyl-2-oxo-1,3-dioxol-4-yl)(lower)alkyl ester [e.g., (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester and the like]; lower alkenyl ester (e.g., vinyl ester, allyl ester and the like); lower alkynyl ester (e.g., ethynyl ester, propynyl ester and the like); ar(lower)alkyl ester optionally having at least one suitable substituent (e.g., benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-t-butylbenzyl ester and the like); aryl ester optionally having at least one suitable substituent (e.g., phenyl ester, 4-chlorophenyl ester, tolyl ester, t-butylphenyl ester, xylyl ester, mesityl ester, cumenyl ester and the like) ; phthalidyl ester; and the like.

Preferable examples of the thus-defined esterified carboxy include lower alkoxycarbonyl and phenyl (or nitrophenyl) (C1-C4)alkoxycarbonyl, with most preference given to methoxycarbonyl, ethoxycarbonyl and benzyloxycarbonyl.

Preferable "amidated carboxy" includes the following.
carbamoyl,
mono or di(lower)alkylcarbamoyl (lower alkyl is as mentioned above) [e.g., methylcarbamoyl, dimethylcarbamoyl, isopropylcarbamoyl, n-butylcarbamoyl, t-butylcarbamoyl, N-methyl-N-(pyridylmethyl)carbamoyl and the like],
aryl(lower)alkylcarbamoyl (aryl and lower alkyl is as mentioned above) [e.g., benzylcarbamoyl, 3,4-methylenedioxybenzylcarbamoyl, diaminobenzylcarbamoyl, phenethylcarbamoyl],
cyclo(lower)alkylcarbamoyl having 3 to 7 carbon atoms (cyclo(lower)alkyl is as mentioned above) [e.g., cyclopropylcarbamoyl, cyclobutylcarbamoyl, cyclopentylcarbamoyl, cyclohexylcarbamoyl and the like],
arylcarbamoyl (aryl is as mentioned above) [e.g., phenylcarbamoyl, naphthylcarbamoyl and the like],
heterocyclic carbamoyl (heterocyclic ring is as mentioned above) [e.g., thiazolylcarbamoyl, thiadiazolylcarbamoyl, pyridylcarbamoyl, triazolylcarbamoyl, tetrazolylcarbamoyl, N-methyl-N-pyridinecarbamoyl, morpholinocarbamoyl and the like],
heterocyclic lower alkylcarbamoyl (heterocyclic lower alkyl is as mentioned above) [e.g., morpholinoethylcarbamoyl, pyridylmethylcarbamoyl, methylenedioxybenzylcarbamoyl and the like],
N-disubstituted carbamoyl wherein nitrogen atom forms a part of nitrogen-containing heterocyclic ring (e.g., morpholinocarbonyl, thiomorpholinocarbonyl, 1-perhydroazevinylcarbonyl, 1,1-dioxothiazolidinecarbonyl, piperidinocarbonyl, 1-piperazinylcarbonyl, 4-(2-hydroxyethyl)-1-piperazinylcarbonyl, 4-methyl-1-piperazinylcarbonyl, carboxypyrrolidinocarbonyl and the like),
substituted sulfonylcarbamoyl and the like.

The substituent of the substituted sulfonylcarbamoyl is exemplified by the aforementioned alkyl having up to 8 carbon atoms, halo(lower)alkyl, aryl(lower)alkyl, hydroxy(lower)alkyl, tri(lower)alkylsilyl(lower)alkyl, lower alkoxy(lower)alkyl, lower alkylthio(lower)alkyl, heterocyclic ring, aryl and the like, wherein aryl may be substituted by the aforementioned halogen atom, lower alkyl, halo(lower)alkyl, lower alkoxy, nitro and the like. Specific examples include naphthylsulfonylcarbamoyl, benzenesulfonylcarbamoyl, nitrobenzenesulfonylcarbamoyl, trihalobenzenesulfonylcarbamoyl, lower alkoxybenzenesulfonylcarbamoyl, halobenzenesulfonylcarbamoyl, mono or di(lower)-alkylbenzenesulfonylcarbamoyl, alkylsulfonylcarbamoyl having 1 to 8 carbon atoms (t-butylsulfonylcarbamoyl, butylsulfonylcarbamoyl, propylsulfonylcarbamoyl, isopropylsulfonylcarbamoyl, methylsulfonylcarbamoyl, octylsulfonylcarbamoyl, pentylsulfonylcarbamoyl, isopentylsulfonylcarbamoyl, hexylsulfonylcarbamoyl and the like), trihalo(lower)alkylsulfonylcarbamoyl, phenyl(lower)alkylsulfonylcarbamoyl, tri(lower)alkylsulfonylcarbamoyl, lower alkylthio(lower)alkylsulfonylcarbamoyl, lower alkoxy(lower)alkylsulfonylcarbamoyl, quinolylsulfonylcarbamoyl and the like.

Preferable "acyl" includes aliphatic acyl, aromatic acyl, heterocyclic acyl and aliphatic acyl substituted by aromatic group or heterocyclic ring, wherein acyl is derived from carboxylic acid, carbonic acid, sulfonic acid, carbamic acid and the like.

This aliphatic acyl includes saturated or unsaturated, acyclic or cyclic one, which is exemplified by alkanoyl such as lower alkanoyl (e.g., formyl, acetyl, propionyl, butyryl, isobutyryl, valeryl, isovaleryl, pivaloyl, hexanoyl etc.) and the like, alkylsulfonyl such as lower alkylsulfonyl (e.g., mesyl, ethylsulfonyl, propylsulfonyl, isopropylsulfonyl, butylsulfonyl, isobutylsulfonyl, pentylsulfonyl, hexylsulfonyl etc.) and the like, carbamoyl, N-alkylcarbamoyl (e.g., methylcarbamoyl, ethylcarbamoyl etc.), alkoxycarbonyl such as lower alkoxycarbonyl (e.g., methoxycarbonyl, ethoxycarbonyl, propoxycarbonyl, butoxycarbonyl, t-butoxycarbonyl etc.) and the like, alkenyloxycarbonyl such as lower alkenyloxycarbonyl (e.g., vinyloxycarbonyl, allyloxycarbonyl etc.) and the like, alkenoyl such as lower alkenoyl (e.g., acryloyl, methacryloyl, crotonoyl etc.) and the like, cycloalkanecarbonyl such as cyclo(lower)alkanecarbonyl (e.g., cyclopropanecarbonyl, cyclopentanecarbonyl, cyclohexanecarbonyl etc.) and the like, and the like.

The aromatic acyl is exemplified by C6-C10 aroyl (e.g., benzoyl, toluoyl, xyloyl and the like), N-(C6-C10) arylcarbamoyl (e.g., N-phenylcarbamoyl, N-tolylcarbamoyl, N-naphthylcarbamoyl and the like), C6-C10 arenesulfonyl (e.g., benzenesulfonyl, tosyl and the like) and the like.

The heterocyclic acyl is exemplified by heterocyclic carbonyl; heterocyclic (lower)alkanoyl (e.g., heterocyclic acetyl, heterocyclic propanoyl, heterocyclic butanoyl, heterocyclic pentanoyl, heterocyclic hexanoyl and the like); heterocyclic (lower)alkenoyl (e.g., heterocyclic propenoyl, heterocyclic butenoyl, heterocyclic pentenoyl, heterocyclic hexenoyl and the like); heterocyclic glyoxyloyl; heterocyclic sulfinyl; heterocyclic sulfonyl; and the like.

The aliphatic acyl substituted by aromatic group is, for example, aralkoxycarbonyl such as phenyl(lower)alkoxycarbonyl (e.g., benzyloxycarbonyl, phenethyloxycarbonyl etc.) and the like.

The acyl may be further substituted by one or more suitable substituents, such as nitro and the like. Examples of preferable acyl having such substituent include nitroaralkoxycarbonyl (e.g., nitrobenzyloxycarbonyl etc.) and the like.

The acylamino means the above-mentioned acyl substituted by amino, such as lower alkylsulfonylamino, lower alkanoylamino, lower alkoxycarbonylamino, ureido, arylsulfonylamino, heterocyclic sulfonylamino and the like.

The neutral substituent may be, for example, halogen atom or alkyl, aralkyl, alkynyl, lower alkoxy and halogen-substituted compounds thereof. When the substituent is hydrocarbon, it may be saturated or unsaturated, and a chain or ring. Further, it may be branched. When it is halogen atom or a halogen-substituted compound, the kind of halogen and the number thereof are not limited.

The position of substitution of R⁴ in the formula (1) is subject to no particular limitation, and it may be at the ortho-position, meta-position or para-position, relative to other substituents.

The preferable salts of the compound of the formulas (1) and (2) of the present invention are nontoxic and pharmaceutically acceptable conventional salts, which are exemplified by salts of alkali metal, such as sodium, potassium and the like, salts of alkaline earth metal, such as calcium, magnesium and the like, salts with inorganic base such as ammonium salt and the like, salts with organic amine, such as triethylamine, pyridine, picoline, ethanolamine, triethanolamine, dicyclohexylamine, N,N'-dibenzylethyleneamine and the like, salts with inorganic acid, such as hydrochloric acid, hydrobromic acid, sulfuric acid, phosphoric acid and the like, salts with organic carboxylic acid, such as formic acid, acetic acid, trifluoroacetic acid, maleic acid, tartaric acid and the like, addition salts of sulfonic acid, such as methanesulfonic acid, benzenesulfonic acid, p-toluenesulfonic acid and the like, and salts and acid addition salts with a base such as basic or acidic amino acid (e.g., arginine, aspartic acid, glutamic acid and the like).

The above-mentioned compounds can be purified as necessary by a conventional purification method of organic compound, such as recrystallization, column chromatography, thin layer chromatography, high performance liquid chromatography and the like. The compound can be identified by NMR spectrum analysis, mass spectrum analysis, IR spectrum analysis, elemental analysis, measurement of melting point and the like.

The compound of the present invention may have one or more asymmetric centers, which can exist as an enantiomer or a diastereomer. Some compounds of the formula containing alkenyl can exist as a cis- or trans-isomer. In any case, the present invention encompasses mixtures thereof and respective isomers.

The compound of the present invention may exist as a tautomer. The present invention encompasses mixtures thereof and such tautomer.

The compound and salts thereof of the present invention may take the form of a solvate, which is also encompassed in the present invention. Examples of the solvate preferably include hydrate and ethanol solvate.

Of the compounds of the formula (1), the compound of the following formula (3), the compound of the formula (4), and the compound of the formula (5) and pharmaceutically acceptable salts thereof are particularly preferable.

### Compound (3)

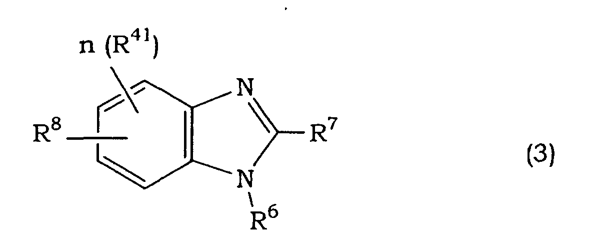

wherein R⁶ is aryl(lower)alkyl optionally substituted by one or two substituents selected from the group consisting of halogen atom, haloaryl, lower alkyl, halo(lower)alkyl, lower alkoxy, nitro, amino, cyano, aryl, cyanoaryl, aryl(lower)alkoxy, arylsulfonyl(lower)alkyl, arylsulfonylamino, aryl(lower)alkyl and heterocyclic ring,
R⁷ is lower alkyl or lower cycloalkyl,
R⁸ is carbamoyl,
wherein the carbamoyl is optionally substituted by lower alkyl optionally substituted by optionally substituted aryl or optionally substituted heterocyclic ring, aryl, heterocyclic ring or the formula (3-a) 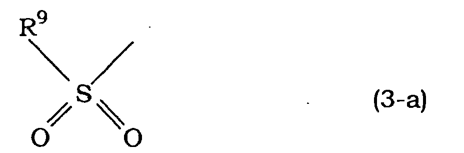
wherein R⁹ is lower alkyl, halo(lower)alkyl, aryl(lower)alkyl, hydroxy(lower)alkyl, tri(lower)alkylsilyl(lower)alkyl, lower alkoxy(lower)alkyl, lower alkylthio(lower)alkyl, heterocyclic ring or aryl, wherein the aryl is optionally substituted by halogen atom, lower alkyl, halo(lower)alkyl, lower alkoxy or nitro, and R⁸ may be bonded to the skeleton via lower alkylene or lower alkenylene,
R⁴¹ is hydrocarbon optionally substituted by halogen, and
n is an integer of 0 - 3.

### Compound (4)

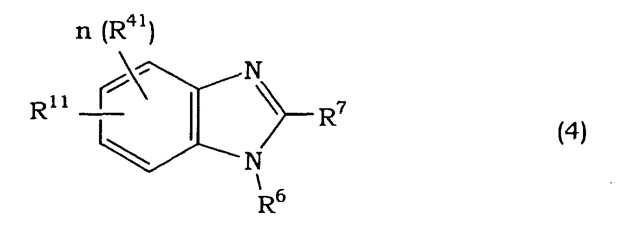

wherein R¹¹ is a substituent of the formula (4-a) 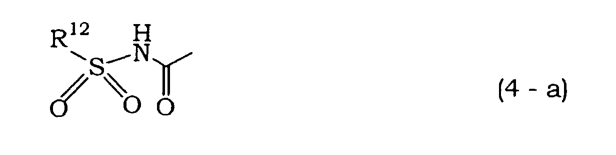 wherein R¹² is lower alkyl, halo(lower)alkyl, aryl(lower)alkyl, hydroxy(lower)alkyl, tri(lower)alkylsilyl(lower)alkyl, lower alkoxy(lower)alkyl, lower alkylthio(lower)alkyl, heterocyclic ring or aryl wherein aryl may be substituted by halogen atom, lower alkyl, halo(lower)alkyl, lower alkoxy or nitro, and R¹¹ may be bonded to the skeleton via lower alkylene or lower alkenylene, and other symbols are as defined above.

### Compound (5)

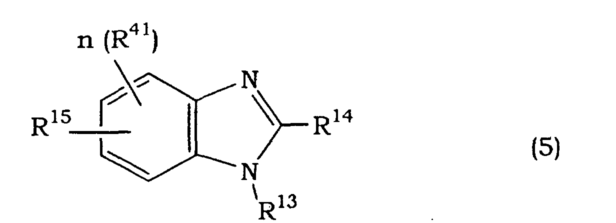

wherein R¹³ aryl(lower)alkyl optionally substituted by one or two substituents selected from the group consisting of halogen atom, lower alkyl, halo(lower)alkyl, lower alkoxy, nitro, amino, cyano, aryl, haloaryl, cyanoaryl, aryl(lower)alkyl, arylsulfonyl(lower)alkyl, arylsulfonylamino and heterocyclic ring,
R¹⁴ is lower alkyl,
R¹⁵ is a substituent of the formula (5-a) 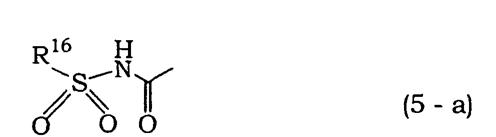
wherein R¹⁶ is lower alkyl or aryl, and other symbols are as defined above.

Of the compounds of the formula (2), the compounds of the following formulas (6) and (7) and pharmaceutically acceptable salts thereof are particularly preferable.

### Compound (6)

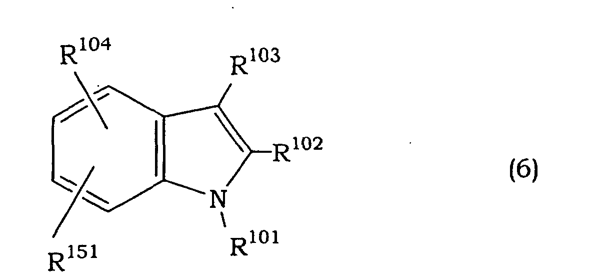

wherein R¹⁵¹ is
   1) carboxy,
   2) esterified carboxy or
   3) the following formula (6-a) 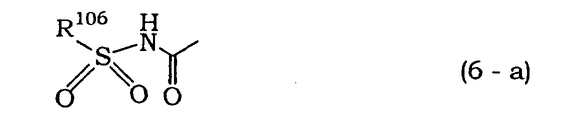
wherein R¹⁰⁶ is
   a) alkyl,
   b) alkenyl,
   c) lower alkoxy(lower)alkyl,
   d) aryl,
   e) heterocyclic ring or
   f) lower cycloalkyl,
   which is optionally substituted by at least one member from halogen atom, lower alkoxy, lower alkyl, lower alkenyl, lower cycloalkyl, nitro, aryl, heterocyclic ring, arylazo, halo(lower)alkyl, lower alkylaryl and lower alkoxyaryl, and nitrogen atom of sulfonamide in the formula (6-a) may leave hydrogen and form a ring with R¹⁰⁶, and other symbols are as defined above.

### Compound (7)

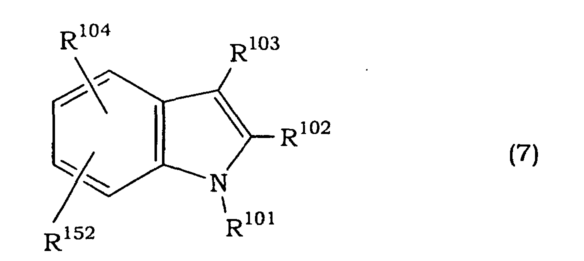

wherein R¹⁵² is a substituent of the formula (7-a) 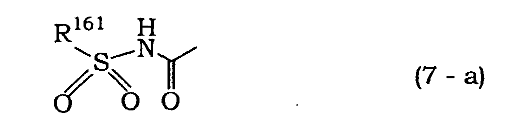 wherein R¹⁶¹ is alkyl, alkenyl, lower alkoxy(lower)alkyl, aryl, heterocyclic ring, lower cycloalkyl, arylalkenyl or heterocyclic alkenyl, and nitrogen atom of sulfonamide in the formula (7-a) may leave hydrogen and form a ring with R¹⁶¹, and other symbols are as defined above.

Of the compounds of the formula (7), the following compounds and pharmaceutically acceptable salts thereof are more preferable.

A compound of the formula (7) wherein R¹⁰¹ is optionally substituted aryl, or lower alkyl, oxy, oxy(lower)alkyl, lower alkoxy, carbonyl, lower alkenyl, optionally substituted imino, lower alkylimino wherein nitrogen atom may be substituted, thio(lower)alkyl or lower alkylthio, which is bonded with or substituted by heterocyclic ring, and
R¹⁰² and R¹⁰³ are hydrogen atoms, lower alkyl, lower alkylthio or lower alkoxy(lower)alkyl.

A compound of the formula (7) wherein R¹⁰² is optionally substituted aryl, or lower alkyl, oxy, oxy(lower)alkyl, lower alkoxy, carbonyl, lower alkenyl, optionally substituted imino, lower alkylimino wherein nitrogen atom may be substituted, thio(lower)alkyl or lower alkylthio, which is bonded with or substituted by heterocyclic ring, and
R¹⁰¹ and R¹⁰³ are hydrogen atoms, lower alkyl, lower alkylthio or lower alkoxy(lower)alkyl.

A compound of the formula (7) wherein R¹⁰³ is optionally substituted aryl, or lower alkyl, oxy, oxy(lower)alkyl, lower alkoxy, carbonyl, lower alkenyl, optionally substituted imino, lower alkylimino wherein nitrogen atom may be substituted, thio(lower)alkyl or lower alkylthio, which is bonded with or substituted by heterocyclic ring, and
R¹⁰¹ and R¹⁰² are hydrogen atoms, lower alkyl, lower alkylthio or lower alkoxy(lower)alkyl.

Specific examples of the compound of the formula (1) include the following:
2-butyl-1-(2-chlorobenzyl)-6-ethoxycarbonylbenzimidazole,
1-(4-bromo-2-fluorobenzyl)-2-butyl-6-ethoxycarbonylbenzimidazole,
2-butyl-1-(2,4-dichlorobenzyl)-6-ethoxycarbonylbenzimidazole,
2-butyl-6-ethoxycarbonyl-1-(4-methoxycarbonylbenzyl)-benzimidazole,
2-butyl-6-ethoxycarbonyl-1-(2-fluorobenzyl)benzimidazole,
2-butyl-6-ethoxycarbonyl-1-(2-trifluoromethylbenzyl)-benzimidazole,
1-(2-chlorobenzyl)-6-ethoxycarbonyl-2-ethylbenzimidazole,
1-(2-chlorobenzyl)-6-ethoxycarbonyl-2-propylbenzimidazole,
1-(2-chlorobenzyl)-2-cyclopropyl-6-ethoxycarbonylbenzimidazole,
1-(2-chlorobenzyl)-6-ethoxycarbonyl-2-isopropylbenzimidazole,
2-butyl-1-(2-chlorobenzyl)-5-ethoxycarbonylbenzimidazole,
2-butyl-1-(2-chlorobenzyl)-7-ethoxycarbonylbenzimidazole,
1-(2-chlorobenzyl)-5-ethoxycarbonyl-2-propylbenzimidazole,
2-butyl-1-(2-chlorobenzyl)-6-carboxybenzimidazole,
2-butyl-6-carboxy-1-(4-carboxybenzyl)benzimidazole,
6-carboxy-1-(2-chlorobenzyl)-2-ethylbenzimidazole,
6-carboxy-1-(2-chlorobenzyl)-2-propylbenzimidazole,
6-carboxy-1-(2-chlorobenzyl)-2-cyclopropylbenzimidazole,
2-butyl-5-carboxy-1-(2-chlorobenzyl)imidazol,
2-butyl-1-(2-chlorobenzyl)-6-dimethylcarbamoylbenzimidazole,
6-(benzylcarbamoyl)-2-butyl-1-(2-chlorobenzyl)benzimidazole,
2-butyl-1-(2-chlorobenzyl)-6-morpholinocarbonylbenzimidazole,
2-butyl-6-carbamoyl-(2-chlorobenzyl)benzimidazole,
2-butyl-1-(2-chlorobenzyl)-6-(4-methylpiperazinyl)-carbonylbenzimidazole,
2-butyl-1-(2-chlorobenzyl)-6-(methylcarbamoyl)benzimidazole,
6-carbamoyl-1-(2-chlorobenzyl)-2-ethylbenzimidazole,
6-carbamoyl-1-(2-chlorobenzyl)-2-propylbenzimidazole,
6-carbamoyl-1-(2-chlorobenzyl)-2-cyclopropylbenzimidazole,
2-butyl-5-carbamoyl-1-(2-chlorobenzyl)benzimidazole,
2-butyl-1-(2-chlorobenzyl)-6-(isopropylcarbonyl)benzimidazole,
1-(2-chlorobenzyl)-6-chloroformyl-2-propylbenzimidazole,
1-(2-chlorobenzyl)-6-(methylcarbamoyl)-2-propylbenzimidazole,
1-(2-chlorobenzyl)-6-(ethylcarbamoyl)-2-propylbenzimidazole,
1-(2-chlorobenzyl)-6-(isopropyl)carbamoyl-2-propylbenzimidazole,
1-(2-chlorobenzyl)-6-(piperidinocarbonyl)-2-propylbenzimidazole,
1-(2-chlorobenzyl)-6-(morpholinocarbonyl)-2-propylbenzimidazole,
1-(2-chlorobenzyl)-6-(2-morpholinoethyl)carbamoyl-2-propylbenzimidazole,
1-(2-chlorobenzyl)-6-[4-(2-hydroxyethyl)piperazinyl]carbonyl-2-propylbenzimidazole,
1-(2-chlorobenzyl)-2-propyl-6-(2-pyridylmethyl)-carbamoylbenzimidazole, and
1-(2-chlorobenzyl)-2-propyl-6-[4-(4-phenyl-1,2,3,6-tetrahydropyridine-1-yl)butyl]carbamoylbenzimidazole, with particular preference given to 1-(2,4-dichlorobenzyl)-2-methyl-6-(1-pentanesulfonylcarbamoyl)benzimidazole.

Specific examples of the preferable compound of the formula (2) are as follows.
6-(benzenesulfonylcarbamoyl)-1-(2-chlorobenzyl)-2-methylindole,
1-(biphenyl-4-ylmethyl)-6-(1-butanesulfonylcarbamoyl)-2-ethylindole,
6-(1-butanesulfonylcarbamoyl)-1-(2,4-dichlorobenzyl)-2-methylindole,
1-(2,4-dichlorobenzyl)-2-methyl-6-(1-pentanesulfonylcarbamoyl)indole,
6-(1-butanesulfonylcarbamoyl)-1-(2,4-dichlorobenzyl)-2-ethylindole,
6-(1-butanesulfonylcarbamoyl)-1-(2,4-dichlorobenzyl)indole,
5-(1-butanesulfonylcarbamoyl)-3-(2,4-dichlorobenzyl)indole,
5-(1-butanesulfonylcarbamoyl)-1-(2,4-dichlorobenzyl)indole,
5-(1-butanesulfonylcarbamoyl)-3-(2,4-dichlorobenzyl)-2-methylindole,
5-butanesulfonylcarbamoyl-3-(2,4-dichlorobenzyl)-1-methylindole,
3-(2,4-dichlorobenzyl)-2-methyl-5-(1-pentanesulfonylcarbamoyl)indole,
6-(1-butanesulfonylcarbamoyl)-3-(2,4-dichlorobenzyl)-2-methylindole,
3-(2,4-dichlorobenzyl)-5-(1-pentanesulfonylcarbamoyl)indole,
3-(2,4-dichlorobenzyl)-2-methyl-5-(1-propanesulfonylcarbamoyl)indole,
3-(2,4-dichlorobenzyl)-2-methyl-5-(1-octanesulfonylcarbamoyl)-indole,
5-(benzenesulfonylcarbamoyl)-3-(2,4-dichlorobenzyl)-2-methylindole,
3-(2,4-dichlorobenzyl)-5-(1-hexanesulfonylcarbamoyl)-2-methylindole,
3-(biphenyl-4-ylmethyl)-2-methyl-5-(1-pentanesulfonylcarbamoyl)indole,
3-(2-chlorobenzyl)-2-methyl-5-(1-pentanesulfonylcarbamoyl)-indole,
5-(1-butanesulfonylcarbamoyl)-3-(2,4-dichlorobenzoyl)-2-methylindole,
3-(2,4-dichlorobenzyl)-2-methyl-5-(3-methyl-1-butanesulfonylcarbamoyl)indole,
3-(2,4-dichlorobenzyl)-5-(2-methoxyethanesulfonylcarbamoyl)-2-methylindole,
3-(4-benzyloxybenzyl)-2-methyl-5-(1-pentanesulfonylcarbamoyl)-indole,
3-(2,4-dichlorobenzyl)-5-(1-pentanesulfonylcarbamoyl)-2-propylindole,
3-(2,4-dichlorobenzyl)-2-ethyl-5-(1-pentanesulfonylcarbamoyl)-indole,
3-(1-bromonaphthalen-2-ylmethyl)-2-methyl-5-(1-pentanesulfonylcarbamoyl)indole,
3-((3-chloropyridin-4-yl)methyl)-2-methyl-5-(1-pentanesulfonylcarbamoyl)indole,
2-methyl-5-(1-pentanesulfonylcarbamoyl)-3-(4-(2-phenylethenyl)benzyl)indole,
3-(2,4-dichlorobenzyl)-5-(ethanesulfonylcarbamoyl)-2-propylindole,
3-(2,4-dichlorobenzyl)-2-methyl-5-(2-thiophenesulfonylcarbamoyl)indole,
3-(2,4-dichlorobenzyl)-5-((4-methoxybenzene)-sulfonylcarbamoyl)-2-methylindole,
5-(benzenesulfonylcarbamoyl)-3-(2,4-dichlorobenzyl)-2-ethylindole,
3-((4-chloroisoquinolin-3-yl)methyl)-2-methyl-5-(1-pentanesulfonylcarbamoyl) indole,
3-((4-bromoisoquinolin-3-yl)methyl)-2-methyl-5-(1-pentanesulfonylcarbamoyl)indole,
3-(2,4-dichlorobenzyl)-2-methyl-5-(1-pent-1-enesulfonylcarbamoyl)indole,
3-(2,4-dichlorobenzyl)-2-methyl-5-(trifluoromethane-sulfonylcarbamoyl)indole,
3-(2,4-dichlorobenzyl)-5-(2,2-dimethylpropanesulfonyl-carbamoyl)-2-methylindole,
3-(2,4-dichlorobenzyl)-2-methyl-5-(8-quinolinesulfonyl-carbamoyl)indole,
3-(2,4-dichlorobenzyl)-2-methyl-5-((2-phenylethane)-sulfonylcarbamoyl)indole,
3-(2,4-dichlorobenzyl)-2-methyl-5-(α-toluenesulfonyl-carbamoyl)indole,
5-cyclohexanesulfonylcarbamoyl-3-(2,4-dichlorobenzyl)-2-methylindole,
5-(3-chloro-1-propanesulfonylcarbamoyl)-3-(2,4-dichlorobenzyl)-2-methylindole,
3-(2,4-dichlorobenzyl)-2-methyl-5-(propanesultam-1-ylcarbonyl)indole,
6-(1-butanesulfonylcarbamoyl)-2-(2,4-dichlorobenzyl)-3-methylindole,
1-(2,4-dichlorobenzyl)-3-methyl-6-(1-pentanesulfonylcarbamoyl) indole,
3-(2,4-dichlorobenzyl)-2-methyl-5-((4-methylbenzene)-sulfonylcarbamoyl)indole,
3-(2,4-dichlorobenzyl)-2-methyl-5-((4-nitrobenzene)-sulfonylcarbamoyl)indole,
5-((4-chlorobenzene)sulfonylcarbamoyl)-3-(2,4-dichlorobenzyl)-2-methylindole,
5-((3-chlorobenzene)sulfonylcarbamoyl)-3-(2,4-dichlorobenzyl)-2-methylindole,
5-((2-chlorobenzene)sulfonylcarbamoyl)-3-(2,4-dichlorobenzyl)-2-methylindole,
3-(2,4-dichlorobenzyl)-5-((4-fluorobenzene)sulfonylcarbamoyl)-2-methylindole,
3-(2,4-dichlorobenzyl)-2-methyl-5-(2-naphthalene)-sulfonylcarbamoyl)indole,
3-(2,4-dichlorobenzyl)-2-methyl-5-(1-naphthalene)-sulfonylcarbamoyl)indole,
3-(2,4-dichlorobenzyl)-2-methyl-5-((2-methylbenzene)-sulfonylcarbamoyl)indole,
3-(2,4-dichlorobenzyl)-5-(2,6-dimethylbenzene)-sulfonylcarbamoyl-2-methylindole,
5-(4-bromobenzene)sulfonylcarbamoyl-3-(2,4-dichlorobenzyl)-2-methylindole,
3-(2,4-dichlorobenzyl)-2-methyl-5-((E)-β-styrenesulfonyl-carbamoyl)indole,
3-(2,4-dichlorobenzyl)-2-methyl-5-((4-vinylbenzene)-sulfonylcarbamoyl)indole,
5-((4-phenylazobenzene)sulfonylcarbamoyl)-3-(2,4-dichlorobenzyl)-2-methylindole,
3-(2,4-dichlorobenzyl)-2-methyl-5-((4-trifluoromethylbenzene)-sulfonylcarbamoyl)indole,
3-(2,4-dichlorobenzyl)-2-methyl-5-((4-methyl-1-pent-1-ene)sulfonylcarbamoyl)indole,
3-(2,4-dichlorobenzyl)-5-((3,4-dimethoxybenzene)-sulfonylcarbamoyl)-2-methylindole,
5-((4-t-butylbenzene)sulfonylcarbamoyl)-3-(2,4-dichlorobenzyl)-2-methylindole,
3-(2,4-dichlorobenzyl)-2-methyl-5-((3-methylbenzene)-sulfonylcarbamoyl)indole,
3-(2,4-dichlorobenzyl)-2-methyl-5-(2-octanesulfonylcarbamoyl)-indole,
3-(2,4-dichlorobenzyl)-2-methyl-5-((4-phenylbenzene)-sulfonylcarbamoyl)indole,
3-((2-chloro-4-phenyl)benzyl)-2-methyl-5-((1-pent-1-ene)-sulfonylcarbamoyl)indole,
3-(2-chloro-4-phenylbenzyl)-2-methyl-5-(1-pentanesulfonylcarbamoyl)indole,
5-(benzenesulfonylcarbamoyl)-3-((2-chloro-4-phenyl)benzyl)-2-methylindole,
3-(2,4-dichlorobenzyl)-5-((4-ethylbenzene)sulfonylcarbamoyl)-2-methylindole,
5-((4-n-butylbenzene)sulfonylcarbamoyl)-3-(2,4-dichlorobenzyl)-2-methylindole,
5-((4-n-butoxybenzene)sulfonylcarbamoyl)-3-(2,4-dichlorobenzyl)-2-methylindole,
3-(2,4-dichlorobenzyl)-2-methylthio-5-((1-pent-1-ene)-sulfonylcarbamoyl)indole,
5-(benzenesulfonylcarbamoyl)-3-(2,4-dichlorobenzyl)-2-methylthioindole,
3-(2,4-dichlorobenzyl)-2-methylthio-5-(1-pentanesulfonyl-carbamoyl)indole,
3-(2,4-dichlorobenzyl)-2-methyl-5-((1-penta-1,3-diene)-sulfonylcarbamoyl)indole,
5-((2-cyclopropylethylene)sulfonylcarbamoyl)-3-(2,4-dichlorobenzyl)-2-methylindole,
3-(2,4-dichlorobenzyl)-2-methyl-5-((4-methyl-(E)-β-styrene)-sulfonylcarbamoyl)indole,
3-(2,4-dichlorobenzyl)-5-((4-methoxy-(E)-β-styrene)-sulfonylcarbamoyl)-2-methylindole,
3-(2,4-dichlorobenzyl)-2-methoxymethyl-5-(1-pentanesulfonyl-carbamoyl)indole,
3- ((1-bromonaphthalen-2-yl)methyl)-2-methyl-5- ((E) -β-styrene)sulfonylcarbamoyl)indole,
3-((1-bromonaphthalen-2-yl)methyl)-2-methyl-5-(4-vinylbenzene)sulfonylcarbamoyl)indole,
3- ((1-bromonaphthalen-2-yl)methyl) -2-methyl-5- (p-toluenesulfonylcarbamoyl)indole,
5-(benzenesulfonylcarbamoyl)-3-((1-bromonaphthalen-2-yl)methyl)-2-methylindole,
3- ((2-chloro-4-phenyl)benzyl)-2-methyl-5-((E)-β-styrenesulfonylcarbamoyl)indole,
3-((2-chloro-4-phenyl)benzyl)-2-methyl-5-((4-vinylbenzene)-sulfonylcarbamoyl)indole,
3-((1-bromonaphthalen-2-yl)methyl) -2-methyl-5-((1-pent-1-ene)sulfonylcarbamoyl)indole,
3-((2-chloro-4-phenyl)benzyl)-2-methyl-5-(p-toluenesulfonyl-carbamoyl)indole,
3-(4-bromo-2-chlorobenzyl)-2-methyl-5-(1-pentanesulfonyl-carbamoyl)indole,
3-(4-bromo-2-chlorobenzyl)-2-methyl-5-(2-(5-chlorothienyl)-sulfonylcarbamoyl)indole,
3-(2-chloro-4-nitrobenzyl)-2-methyl-5-(1-pentanesulfonyl-carbamoyl)indole,
3-(2-chloro-4-(2-phenylethenyl)benzyl)-2-methyl-5-(1-pentanesulfonylcarbamoyl)indole and the like.

The above-mentioned compounds of the formulas (1) and (2) and pharmaceutically acceptable salts thereof to be used in the present invention are particularly useful for the treatment and prophylaxis of polycystic ovary syndrome, based on the insulin resistance-improving action. Besides this, for example, they are also useful for the treatment and prophylaxis of gestational diabetes, diabetic complication (diabetic osteopenia, diabetic osteoporosis), autoimmune disease, pancreatitis, and cachexia (e.g., progressive weight loss due to lipolysis, myodegeneration, anemia, edema, anorexia and the like in chronic diseases such as cancer, tuberculosis, endocrine disease, AIDS and the like).

When the compound of the formula (1) or (2) is used for treatment, it is admixed with a pharmaceutically acceptable carrier suitable for oral administration, parenteral administration or external application (topical application), such as a solid, semi-solid or liquid, organic or inorganic excipient, and used in the form of a typical pharmaceutical preparation containing the aforementioned compound as an active ingredient. The pharmaceutical preparation may be a solid such as tablet, granule, powder, capsule, dragee and suppository, a liquid such as solution, suspension, milky liquid, syrup, emulsion, lemonade, lotion and the like, or an ointment or gel. Where necessary, the above-mentioned preparation may contain conventional additives such as auxiliaries, stabilizer, humectant, adjuvant, emulsifier, buffer and the like.

While the dose of compounds (1) and (2) varies depending on the age and conditions of patients, an effective amount of the compound (1) or (2) for the treatment of the above-mentioned diseases would be about 0.1 mg, 1 mg, 10 mg, 50 mg, 100 mg, 250 mg, 500 mg or 1000 mg on average for each dose. In general, a dose of 0.1 mg/individual - about 1000 mg/individual is administered per day.

The present invention is explained in more detail in the following by referring to Examples. The present invention is not limited to these examples.

### Example 1

### Production of 1-(2,4-dichlorobenzyl)-2-methyl-6-(1-pentanesulfonylcarbamoyl)benzimidazole

### Step 1

### Production of ethyl 4-amino-3-nitrobenzoate

4-Amino-3-nitrobenzoic acid (200.0 g) was suspended in ethanol (1030 g). Thereto was added dropwise conc. sulfuric acid (120.0 g) over 0.5 hr, and after the dropwise addition, the mixture was refluxed under heating for 15 hr. A part (422 g) of ethanol was distilled away at atmospheric pressure, and water (660 g) was added dropwise to allow precipitation of crystals. To this suspension were added 25% aqueous sodium hydroxide solution (50 g) and then 2.5% aqueous sodium hydroxide solution (262 g), and pH was adjusted to 6.5. The precipitated crystals were collected by filtration, washed successively with ethanol (531 g) and water (1069 g), and dried under reduced pressure to give ethyl 4-amino-3-nitrobenzoate (200.6 g).
¹H-NMR (CDCl₃ δ ppm): 1.39(3H, t, J=7.1Hz), 4.37(2H, q, J=7.1Hz), 6.41(2H, brs), 6.83(1H, d, J=8.7Hz), 8.00(1H, dd, J=1.9 and 8.7Hz), 8.85(1H, d, J=1.9Hz)

### Step 2

### Production of ethyl 4-(acetylamino)-3-nitrobenzoate

To a mixture of ethyl 4-amino-3-nitrobenzoate (142 g), dimethylaniline (110 ml) and toluene (940 ml) was added dropwise acetyl chloride (62 ml) in an ice bath. The mixture was stirred at 50°C for 3 hr and cooled, and water (142 ml) was added to stop the reaction. The toluene layer was separated, and the organic layer was washed with dil. hydrochloric acid and then with water. The organic layer was concentrated to about 1/3 in volume, and hexane (284 ml) was added to allow crystallization. The crystals were filtrated and washed with hexane to give ethyl 4-(acetylamino)-3-nitrobenzoate (157.7 g).
¹H-NMR (CDCl₃ δ ppm): 1.42(3H, t, J=7.1Hz), 2.33(3H, s), 4.42(2H, q, J=7.1Hz), 8.28(1H, dd, J=2.1 and 8.9Hz), 8.89(1H, d, J=2.1Hz), 8.91(1H, d, J=8.9Hz), 10.55(1H, brs)

### Step 3

### Production of ethyl 4-(acetylamino)-3-aminobenzoate

A mixture of ethyl 4-(acetylamino)-3-nitrobenzoate (152 g), ethanol (1400 ml) and palladium on carbon (carrying rate 5%, 15 g) was stirred at room temperature under a hydrogen atmosphere for 45 hr. Ethanol (1000 ml) and methanol (1500 ml) were added to dissolve the precipitated crystals and palladium on carbon was filtered off. The filtrate was concentrated to 724 g and ice-cooled. The precipitated crystals were collected. The filtrate was concentrated to about 1/2 to collect the precipitated crystals. The crystals were combined and dried under reduced pressure to give ethyl 4-(acetylamino)-3-aminobenzoate (105 g).
¹H-NMR (DMSO-d₆ δ ppm): 1.28(3H, t, J=7.1Hz), 2.06(3H, s), 4.24(2H, q, J=7.1Hz), 5.19(2H, s), 7.14(1H, dd, J=1.9 and 8.3Hz), 7.35(1H, d, J=1.9Hz), 7.48(1H, d, J=8.3Hz), 9.19(1H, s)

### Step 4

### Production of ethyl 4-(acetylamino)-3-((2,4-dichlorobenzyl)-amino)benzoate

A mixture of ethyl 4-(acetylamino)-3-aminobenzoate (250 g), 2,4-dichlorobenzyl chloride (264 g), potassium carbonate (187 g), sodium iodide (50.6 g), water (500 g) and ethyl acetate (1100 g) was stirred at 70 - 73°C for 16 hr. The ethyl acetate layer was separated while the mixture was hot, and t-butyl methyl ether (1500 g) was added. The reaction mixture was cooled to 10°C, and the precipitated crystals were filtrated and washed with a mixed solvent (1/2, 600 g) of ethyl acetate and t-butyl methyl ether. The crystals were dried to give ethyl 4-(acetylamino)-3-((2,4-dichlorobenzyl)amino)benzoate (296.6 g).
¹H-NMR (CDCl₃ δ ppm): 1.36(3H, t, J=7.1Hz), 2.23(3H, s), 4.32(2H, q, J=7.1Hz), 4.35-4.48(3H, m), 7.20(1H, d, J=8.2Hz), 7.32(1H, d, J=8.3Hz), 7.38(1H, s), 7.42(2H, d, J=2.0Hz), 7.46(1H, d, J=8.2Hz), 7.50(1H, d, J=8.2Hz)
melting point: 158.2 - 159.9°C

### Step 5, Step 6

### Production of 6-carboxy-1-(2,4-dichlorobenzyl)-2-methylbenzimidazole

Ethyl 4-(acetylamino)-3-((2,4-dichlorobenzyl)amino)benzoate (250 g) was dissolved in conc. hydrochloric acid (68.4 g) and ethanol (1500 ml), and stirred at 72 - 78°C for 2 hr to allow conversion to 1-(2,4-dichlorobenzyl)-6-(ethoxycarbonyl)-2-methylbenzimidazole. To this reaction mixture was added a solution of sodium hydroxide (105 g) and water (1000 ml), and the mixture was stirred at 73 - 77°C for 2 hr. While cooling the reaction mixture, conc. hydrochloric acid (193 g) was added at not more than 35°C and the pH of the solution was adjusted to 6. The mixture was stirred at 20°C for 1 hr, and the precipitated crystals were collected and washed with a mixture of ethanol (600 ml) and water (300 ml). The crystals were dried to give 6-carboxy-1-(2,4-dichlorobenzyl)-2-methylbenzimidazole (214 g). 1-(2,4-dichlorobenzyl)-6-(ethoxycarbonyl)-2-methylbenzimidazole ¹H-NMR (CDCl₃ δ ppm) : 1.40(3H, t, J=7.2Hz), 2.57(3H, s), 4.38(2H, q, J=7.2Hz), 5.41(2H, s), 6.34(1H, d, J=8.4Hz), 7.09(1H, dd, J=2.2 and 8.4Hz), 7.50(1H, d, J=2.2Hz), 7.76(1H, d, J=8.3Hz), 7.91(1H, d, J=1.3Hz), 8.00(1H, dd, J=1.5 and 8.5Hz) 6-carboxy-1-(2,4-dichlorobenzyl)-2-methylbenzimidazole
¹H-NMR (DMSO-d₆ δ ppm): 2.51(3H, s), 5.61(2H, s), 6.53(1H, d, J=8.4Hz), 7.32(1H, dd, J=8.4 and 2.0Hz), 7.64(1H, d, J=8.4Hz), 7.73(1H, d, J=2.1Hz), 7.80(1H, dd, J=8.4 and 1.5Hz), 7.97(1H, s), 12.71(1H, brs)
melting point: 310.1 - 311.2°C

### Step 7

### Production of 1-(2,4-dichlorobenzyl)-2-methyl-6-(1-pentanesulfonylcarbamoyl)benzimidazole

A mixture of 6-carboxy-1-(2,4-dichlorobenzyl)-2-methylbenzimidazole (130 g), N,N'-carbodiimidazole (81.8 g) and N,N-dimethylformamide (1300 g) was stirred at room temperature for 1 hr. 1-Pentanesulfonamide (76.2 g) and 1,8-diazabicyclo[5.4.0]undec-7-ene (76.8 g) were added and the mixture was stirred at 90°C for 15 hr. The reaction mixture was concentrated to 490 g under reduced pressure, and methanol (1300 ml) and water (650 ml) were added. While cooling the solution to not more than 30°C, conc. hydrochloric acid (142 g) was added dropwise to neutralize the solution to pH 5, and the mixture was stirred at 20°C for 1.5 hr. The precipitated crystals were collected and washed with a mixture of methanol (400 ml) and water (200 ml). The crystals were dried to give a crude purification product of 1-(2,4-dichlorobenzyl)-2-methyl-6-(1-pentanesulfonylcarbamoyl)benzimidazole (163 g). This crude purification product (135 g) was dissolved in a mixture of acetone (1620 ml) and water (160 ml) at 60°C. Water (920 g) was gradually added dropwise over 45 min at 60°C, and the mixture was stirred for 14 hr while cooling the mixture to room temperature. The crystals were filtrated, washed with a mixture of acetone (420 ml) and water (280 ml), and dried to give 1-(2,4-dichlorobenzyl)-2-methyl-6-(1-pentanesulfonylcarbamoyl)-benzimidazole (125 g).
¹H-NMR (DMSO-d₆ δ ppm) : 0.81(3H, t, J=7.3Hz), 1.22-1.30(2H, m), 1.32-1.39(2H, m), 1.64-1.71(2H, m), 2.50(3H, s), 3.50(2H, t, J=7.8Hz), 5.59(2H, s), 6.45(1H, d, J=8.4Hz), 7.33(1H, dd, J=2.2 and 8.5Hz), 7.69(1H, d, J=8.5Hz), 7.76(1H, d, J=2.1Hz), 7.80(1H, dd, J=1.6 and 8.5Hz), 8.10(1H, s), 11.89(1H, s)
¹H-NMR (CD₃OD δ ppm): 0.80(3H, t, J=7.3Hz), 1.25(2H, m), 1.34(2H, m), 1.71(2H, m), 2.52(3H, s), 3.43(2H, t), 5.53(2H, s), 6.49(1H, d, J=8.4Hz), 7.14(1H, d, J=8.4Hz), 7.50(1H, s), 7.62(1H, d, J=8.2Hz), 7.76(1H, d, J=8.3Hz), 7.90(1H, s)
IR (Nujol): 1674 cm⁻¹
melting point: 211.7 - 212.5°C

The compound obtained in the above-mentioned Example 1, Step 7 was evaluated to be a drug acting on insulin in blood and blood glucose of ob/ob mice. Experimental method

### 1. Animal

Used were 8 to 10-week-old female C57BL/6J-ob/ob mice (disease group) and C57BL/6J-?/+ mice (normal control group). During the period of acclimation (from purchase to 8 weeks of age when the test was started) and during the experiment, the mice were allowed to freely take a solid feed (CE-2) and water.

### 2. Test drug

The drug is suspended in 0.5% methylcellulose and orally administered once a day (evening). The amount to be administered is 5 ml/kg. The mice are divided into groups, and weighed every week. According to the obtained weight, the amount to be administered is determined.

### 3. Grouping

The mice are weighed and the blood is drawn when the test is started. The plasma concentrations of insulin, glucose and triglyceride are measured. The mice are grouped according to the weight and plasma concentrations of insulin, glucose and triglyceride, as in the following.

The individuals that showed drastically different values from the average values are first excluded to select only the standard level individuals (about 90% of the mice can be used). Then, the selected individuals are grouped to make the average values of the insulin concentration, plasma concentration of glucose, weight and plasma concentration of triglyceride almost constant among the groups.

### 4. Experiment

Two weeks of from 8 to 10 weeks of age are the test period. After the grouping, the drug is consecutively administered orally for 14 days and the blood is drawn. The plasma concentrations of insulin and glucose are measured. The blood is drawn without fasting, starting from after 8:30 A.M. Using heparinized capillary, two capillaries of blood is drawn from the orbital venous plexus. The capillary after drawing the blood is ice-cooled and immediately centrifuged to separate the plasma.

The plasma glucose and triglyceride levels are measured using 10 µl of the plasma by mutarotase-glucose oxydase method and GPQ-DAOS method, respectively (used are Measurement kit: glucose CII-Test Wako and triglyceride E-Test Wako, Wako Pure Chemical Industries, Ltd.). The insulin concentration in plasma is measured using 20 µl of the plasma by the RIA method (kit: Phadeseph insulin, Pharmacia). The plasma glucose and triglyceride levels are measured within the day of drawing blood. The insulin can be preserved upon freezing (-20°C).

The results are shown in Table 1.

**Table 1**

| | Amount administered (mg/kg) | n | Plasma glucose (mg/dl) | Insulin (µU/ml) |
|---|---|---|---|---|
| ob/ob control | | 15 | 204.2±16.8* | 161.0±20.7* |
| Test compound | | | | |
| | 0.3 | 10 | 145.8±18.2* | 110.2±25.3 |
| | 1 | 10 | 142.9±10.6* | 70.9± 9.7** |
| | 3 | 9 | 129.3±10.7** | 50.6± 9.1** |

| | | | | |
|---|---|---|---|---|
| * : p<0.05, | | | | |
| **: p<0.01 vs ob/ob Control, Dunnett's multiple comparison test | | | | |

### Industrial Applicability

The pharmaceutical composition of the present invention, which contains a compound of the formula (1) or (2), or a pharmaceutically acceptable salt thereof as an active ingredient, shows a higher level of the effect of the insulin resistance-improving action than it is in conventional compositions, and therefore, is useful as a therapeutic agent for polycystic ovary syndrome.

This application is based on application No. 324026/1998 filed on November 13, 1998 in Japan, the contents of which are incorporated hereinto by reference.

## Claims

1. A therapeutic agent for polycystic ovary syndrome, which comprises a compound of the following formula (1) or (2), or a pharmaceutically acceptable salt thereof as an active ingredient: 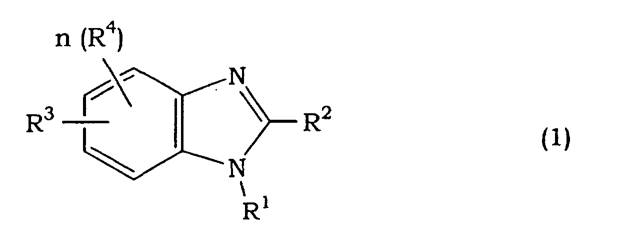 wherein
R¹ is hydrogen atom, arylsulfonyl or lower alkyl wherein lower alkyl may be substituted by one or two group(s) from aryl optionally substituted by a group selected from the group consisting of halogen atom, haloaryl, lower alkyl, halo(lower)alkyl, lower alkoxy, nitro, amino, cyano, aryl, aryl(lower)alkyl, aryl(lower)alkoxy, haloaryl(lower)alkoxy, arylsulfonyl(lower)alkyl, arylsulfonylamino, cyanoaryl and heterocyclic ring, and heterocyclic ring
R² is hydrogen atom, lower cycloalkyl, hydroxy, lower alkoxy, mercapto, lower alkylthio, amino, lower alkylamino, carboxy, aryl or lower alkyl, wherein lower alkyl may be substituted by halogen atom, lower alkoxy, cyano, chlorocarbonyl, aryl or heterocyclic ring.
R³ is carboxy, esterified carboxy, amidated carboxy, amino, amide or sulfonyl, wherein amino and amide are each optionally substituted by acyl or sulfonyl, said sulfonyl being bonded with halogen atom, amino or acylamino and R³ may be bonded to the skeleton via lower alkylene or lower alkenylene, and
R⁴ is a neutral substituent, and
n is an integer of 0 - 3; 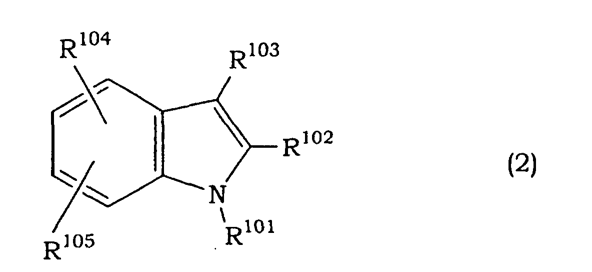 wherein
R¹⁰¹ - R¹⁰³ are each
1) hydrogen atom,
2) lower alkyl, lower alkylthio or lower alkoxy(lower)alkyl or
3) lower alkyl, oxy, oxy(lower)alkyl, lower alkoxy, carbonyl, lower alkenyl, optionally substituted imino, lower alkylimino wherein nitrogen atom is optionally substituted, thio(lower)alkyl or lower alkylthio, wherein each group is bonded with aryl or heterocyclic ring or substituted with aryl or heterocyclic ring,
said aryl and heterocyclic ring being each optionally substituted by halogen atom; nitro; lower alkylamino; acylamino; lower alkyl; lower alkoxy; halo(lower)alkyl; lower cycloalkyl; or aryl, heterocyclic ring, aryl(lower)alkyl, heterocyclic lower alkyl, aryl(lower)alkoxy, heterocyclic lower alkoxy, aryl(lower)alkenyl or heterocyclic lower alkenyl, each being optionally substituted by halogen atom or lower alkyl,
provided that R¹⁰¹ - R¹⁰³ are not hydrogen atoms at the same time,
R¹⁰⁴ is hydrogen atom or lower alkyl, and
R¹⁰⁵ is carboxy, esterified carboxy or amidated carboxy.

2. The therapeutic agent for polycystic ovary syndrome according to claim 1, wherein the active ingredient is 1-(2,4-dichlorobenzyl)-2-methyl-6-(1-pentanesulfonylcarbamoyl)-benzimidazole.
